# EUROPEAN PATENT APPLICATION

(11) **EP 0 894 499 A1**
(43) Date of publication of application: **03.02.1999**
(21) Application number: 97305849.8
(22) Date of filing: 01.08.1997
(51) Int. Cl.: A61K 35/78

(54) **Herbal anti-diabetic composition**

(71) Applicant: Gem Energy Industry Limited, Chennai-600086 (IN)
(72) Inventor: Neelakantan, Kamaswaran, T. Nagar, Chennai 600 017 (IN)
(74) Representative: Skailes, Humphrey John

(57) **Abstract**

A herbal anti-diabetic therapeutic product comprising the powdered inner seed of Dolichos biflorous and the powdered fibrous outer shell of the seed of Dolichos biflorous, wherein the product is usually subjected to (e.g. infra red) radiation.

## Description

This invention relates to a herbal product for use in the treatment of diabetes and its preparation, and in particular to the isolation of an active principle from Dolichos biflorous useful for controlling and reducing diabetes.

Diabetes mellitus is a chronic hereditary disease which is characterized by abnormally high level of glucose in the blood and in the excretion of the urine. It is a disorder of carbohydrate metabolism.

Diabetes occurs at every age and in every race and is never cured, although it may become submerged. It is an universal disease existing in certain geographical, environmental and socioeconomic conditions although there are variations in the pattern of the disease in different parts of the world.

The symptoms of diabetes fall into two main categories. Rapid epinephrine release causes sweating, tremor, tachycardia, anxiety and hunger. Central nervous system symptoms include dizziness, headache, clouding of vision, blunted mental acuity, confusion, abnormal behaviour, convulsions and loss of consciousness. When hypoglycaemia is recurrent or severe, nervous system symptoms predominate, and the epinephrine phase may not be recognizable. With more rapid drops or wide swings in plasma glucose, adrenergic symptoms are prominent (Harrison's Principles of Internal Medicine, 11th Ed., McGraw-Hill Book Company, New York, 1987, p 1800).

Numerous strategies have been developed to achieve the goal of controlling the diabetes such as open looped continuous subcutaneous pumps and multiple daily injections of insulin. With this continuous treatment the patients have to measure their sugar level to ensure that close to normal blood sugar levels are maintained.

The treatment of diabetes was revolutionized with the discovery of insulin in 1921 and further modified after the introduction of oral drugs. However it has been found that the intensive insulin treatment, while it markedly delays and lessens long term retinal, nephrologic and neuropathic disease, leads to a three to nine-fold increase in hypoglycaemic events, most of which occur at night (L. Y. Dawson, Clinical Diabetes, 11:88-96,1993). Sometimes it can lead to loss of consciousness and convulsions. It has been found that severe hypoglycaemic events seem to occur more often at night while the patient is asleep rather then during the day. When awake, diabetic patients can feel hypoglycaemic reactions beginning, and can treat themselves with sugar in order bring their blood sugar levels back into the normal range. When asleep, patients do not have this awareness, and therefore the risk of hypoglycaemia is much higher during this time.

The use of cornstarch to combat hypoglycaemia has also been tried. It has been found that protection against low blood sugar was provided for up to 6 to 8 hours after ingestion of uncooked starch (J. I. Wolfsdorf et al. Am. J. Clin. Nutr., 51:1051-7, 1990). However, the dosage of cornstarch used for this treatment was 1.75 grams per kilogram of body weight. This dosage is much higher than could be tolerated by a patient with diabetes mellitus. It has been found that the cornstarch did little to prevent hypoglycaemia( M. T. Ververs et al., Eur. J. Clin. Nutr., 47:268-73, 1983).

Thus the need exists for a better method for treating hypoglycaemia and a product overcoming the above-mentioned drawbacks, and we have found that blood sugar levels in patients with diabetes mellitus can be regulated and controlled by ingesting a therapeutic herbal (food) composition.

Plants are part and parcel of human society from the dawn of civilization and Dolichos biflorous has been extensively used as a food for both animal and human beings. It is used for example as a feed for cattle and horses. The seeds are cooked before feeding. Stems, leaves and split husk are also used as cattle feed.

The seeds are consumed by the poorer people after cooking or frying. They are eaten whole or after grinding into a meal, unlike other pulses which are consumed after splitting.

Diet has always been considered the most important factor in the management of diabetes even though insulin may be life saving for some.

However the diabetes diet is no way different from the normal diet but for some restriction and modifications. The major principle is to modify the caloric intake on the basis of the height, weight and sex of the individual.

The dietary advice with regard to carbohydrate intake is concerned only with the amount or proportion of simple and complex carbohydrates. In fact both the type and form of carbohydrate have varying effects on blood sugar response.

It has been found that the complex carbohydrates or starches such as rice or potato are slowly digested and absorbed, causing only a small rise in blood glucose level. On the other hand simple carbohydrates such as glucose, lactose and the like , were assumed to be readily digested and absorbed and to produce large and rapid increases in blood glucose levels. Hence diabetics are advised to avoid taking simple sugars.

Diabetes is primarily a disorder of carbohydrate metabolism, as stated earlier. The real danger of diabetes does not however result in from impaired carbohydrates metabolism but from impaired fat metabolism which follows disturbances of the carbohydrate metabolism.

The source of carbohydrates in the diet has a significant influence on lipid(fat) metabolism in human being. As the amount of sucrose is increased and the quantity of complex carbohydrates is decreased, the concentration of cholesterol and more particularly of triglycerides increases. There is a correlation between blood lipid concentration and impaired glucose tolerance.

There is an urgent need to develop strategies to diminish diabetes. It is very important to have an aggressive therapy of prime importance for the prevention and control of diabetes. The main object of the present invention is to provide therapy to maintain a prolong, healthy, productive and successful life.

A further object of the present invention is to provide a product with a shelf life of more than a year.

One of the important objects of the present invention is that it is a 100% herbal medicine and no chemicals are used even as preservatives to increase the shelf life of the said invention. Only the naturally occurring constituents of the composition act as preservatives.

One of the other objects of the present invention is that it is completely anti-toxic. Another objective of the present invention is to treat the defect in metabolism and to preserve pancreatic function to set right the chronic diabetes conditions and promote psychological adjustments.

A study was conducted to determine the effect of present medicine as a diabetic food on the blood glucose, serum cholesterol and triglyceride levels of newly detected diabetes. The therapeutic product was administered as part of an overall programme of treatment, including control of diet. The result was found to be very effective with no side effects.

The present invention concerns a therapeutic product processed from a plant from the family leguminosae whose fibres have an effect on blood sugar level by increasing the viscosity of the unstirred layer between food and the surrounding surface, thereby making the carbohydrates available for absorption at a slower rate.

The herb Dolichos biflorous which is taken from the family leguminosae comprises naturally occurring essential components such as woody fibre (7.5%) and digestible carbohydrate (12.9%). It is a valuable protein supplement, where the seed contains 11.8% moisture, 22.0% crude protein, 0.5% fat, 3.1% mineral matter, 5.3% fibre, 57.3% carbohydrates, 0.28% calcium, 0.39% phosphorous and iron 7.6mg, nicotinic acid 1.5mg and carotene 119mg per 100g.

The part of the herb used in the invention is the seed, which contains albuminoids, starch, oil fibre, ash, phosphoric acid and enzymes. A typical analysis is:
The moisture is normally 4.30 to 10.25%
Ether extract: 0.65 to 1.84%
Albuminoids: 20.75% to 22.25% (containing nitrogen 3.32 to 3.56%)
Soluble carbohydrates: 56.04% to 63.20%
Woody fibre: 4.85% to 5.50%
Ash: 4.20% to 7.45% (containing sand 0.72% to 1.70 respectively)
   (Percentages herein are by weight)

The sugar level in patients with diabetes mellitus is regulated and reduced by ingesting a therapeutic herbal composition including a slowly metabolized complex carbohydrate.

The present therapeutic herbal product is prepared by the processing of the seeds.

The present invention relates to a herbal antidiabetic composition comprising the powdered inner seeds of Dolichos biflorous (preferably from 90-98.5%) and the powdered fibrous outer shells of the seeds of Dolichos biflorous (preferably from 10-1.5%). The product is preferably subjected to radiation, for example, infra red radiation.

The process for the manufacture of this novel product may comprise the steps of:
- First, cleaning of seeds by conventional means to remove the dust and other superfluous particles, e.g. by washing the seeds with water.
- Drying the seeds, for example, first in an open atmosphere at ambient temperatures and then in open pans 45-50°C.
- The drying process results in the colour of seeds changing from brown to golden brown.
- Cooling the seeds to ambient temperature.
- Decortication of the shells: the shells are removed from the said seeds by conventional means and are separated.
- Crushing the seeds: The seeds without shells are crushed in a pulverizer e.g. to 200-300 sieve mesh size. The crushed seeds have an 18% natural protein content.
- Crushing the shells: The crushing of the outer shells is e.g. to 150-200 sieve mesh size which maintains the fibrous strength and length.
- Mixing of the seed powder with the shell powder: The mixing may be done in a conventional mechanical mixer at low RPM (35-60) for half an hour.
- Treatment of mixture: The mixture may be placed in a stainless steel air tight compartment and exposed to infra red rays e.g for 10-20 minutes.
- Packing of the said product.

The normal dose of the herbal anti-diabetic medicine is taken 1-2 teaspoon(s) full (5-10g) of powder. The said mixture can be taken with any liquid or solid diet along with the food. The fibres of the mixture makes a sheath in the intestine to reduce the absorption of the carbohydrates and natural insulin into the blood stream. This enables the patient to sustain energy for a period of 3 to 4 hours at least.

The product of the invention, being a fibrous product, is also useful in curing constipation, as the fibres are indigestible, as they clear up the intestine as they move through it. By the use of conventional medicines, the left-over insulin in the pancreas is comparatively quickly absorbed into the blood stream and energy is only sustained for approximately one hour.

### EXAMPLE 1

The seed of Dolichos biflorous is used for the preparation of an anti-diabetic therapeutic product in the ratio of:
- from about 90.5-98.% of powdered inner seed of Dolichos biflorous
- from about 9.5-2% of powdered fibrous outer shell of the seed of said Dolichos biflorous
   where the seeds are washed with water to remove the dirt or other foreign particles, followed by drying the seeds by heating them up to 40-55°C in open pans. The seeds are allowed to cool to ambient temperature. The outer shell is removed and the decorticated inner seed is ground to a mesh size of 150-250 followed by the crushing of the outer fibrous shell to a sieve size of 100-200 mesh without destroying the length of the fibres. The powdered inner seeds and outer shells are mixed in the ratio 98:2 and exposed to infra-red rays for 10-20 minutes before packing.

The herbal anti-diabetic medicine is taken in an amount of 1-2 teaspoon(s) full of powder, e.g. with any liquid or solid diet along with other food.

### EXAMPLE 2

The seed of Dolichos biflorous is used for the preparation of an anti-diabetic therapeutic product in the ratio of:
- from about 89.5-97.5.% of powdered inner seed of Dolichos biflorous
- from about 10.5-2.5% of powdered fibrous outer shell of the seed of said Dolichos biflorous
   where the seeds are washed with water to remove the dirt or other foreign particles followed by drying by heating at 40-55°C in open pans. The seeds are allowed to cool to ambient temperature. The outer shells are removed and the decorticated inner seeds are grounded to the mesh size of 175-250 followed by the crushing of the outer fibrous shell to a sieve size of 150-250 mesh without destroying the length of the fibres. The powdered inner seeds and outer shells are mixed in the ratio 97.5:2.5 and exposed to infra-red rays for 10-20 minutes before packing.

The herbal anti-diabetic medicine can be used in the same way as for Example 1.

### ADVANTAGES OF THE PRESENT INVENTION

1) The therapeutic medicinal product is the result of composition of naturally occurring non-synthetic plant, having no side effects. No chemical is used for the preservation or synthesis of the end product.
2) One of the components of the product is a high fibre food, which results not only in the reduction of natural insulin level but also helps in treating the constipation.
3) The plant is cultivated in abundance at low cost and is affordable to everybody.
4) The product is a completely herbal product where no chemicals are used even as a preservatives, as the active constituent itself acts as a preservative.
5) Besides having no side effects the product conserves the carbohydrate consumption thus giving the patient more energy with minimum consumption of carbohydrate.

## Claims

1. A herbal anti-diabetic composition which comprises both the powdered inner seed of Dolichos biflorous and the powdered fibrous outer shell of the seed of Dolichos biflorous.

2. A composition as claimed in claim 1 which has been subjected to radiation.

3. A composition as claimed in claim 1 or claim 2 which contains 90-98.5% of the powdered inner seed and 10 to 1.5% of the powdered fibrous outer shell.

4. A composition as claimed in any preceding claim, wherein powdered inner seed has a 200-300 sieve mesh size.

5. A composition as claimed in any preceding claim, wherein the outer fibrous shell has a 150-200 sieve mesh size.

6. A composition as claimed in any preceding claim, wherein the product has been exposed to infra-red radiation.

7. A process for the manufacture of the herbal anti-diabetic composition which comprises cleaning the seeds of Dolichos biflorous, followed by drying the seeds at ambient temperature and then at 40-55°C, cooling the seeds to ambient temperature, decortication of the outer shells of the seeds, crushing the seeds, crushing the shells to a powder size while maintaining the fibrous structures, mixing the powders of the seeds and shells and exposing the mixture to radiation.
